# EUROPEAN PATENT APPLICATION

(11) **EP 1 666 016 A1**
(43) Date of publication of application: **07.06.2006**
(21) Application number: 04727410.5
(22) Date of filing: 14.04.2004
(51) Int. Cl.: A61H 7/00

(54) **TREATMENT APPARATUS**

(30) Priority: 12.09.2003 JP 2003322186
(71) Applicant: YA-MAN LTD, Koto-ku, Tokyo 135-0045 (JP)
(72) Inventor: YAMAZAKI, Iwao, c/o YA-MAN LTD., Tokyo 1350045 (JP)
(74) Representative: Maury, Richard Philip
(86) International application number: PCT/JP2004/005342
(87) International publication number: WO 2005/025478

(57) **Abstract**

A treatment device of the present invention includes: a housing in which an electric component such as a rechargeable battery is incorporated; first and second brush units each having a plurality of brushes and formed of a translucent material; an LED lamp incorporated in a base end part of the plural brushes in the housing; waterproofing O-ring and packing which seal an accommodating portion for the electric component in the housing against the outside; and a brush drive mechanism which reciprocates the first and second brush units in a direction along a surface of a scalp in a manner that approach or separation between tip parts of the plural brushes tip portions of the which are contacted with the scalp is repeated.

## Description

### Technical Field

The present invention relates to a treatment device capable of performing massage for a body part such as a hand/leg, shoulder or the like or performing treatment for hair growth or the like.

### Background Art

Conventionally, a hairbrush is known which is constructed such that the hairbrush can effectively perform hair growth treatment by vibrating a brush part thereof at a time of brushing (for example, see Japanese Patent Laid-open Application No. Hei 10-327936).

That is, this hairbrush device is provided with a motor inside a housing in which brushes are implanted and performs hair growth treatment by vibrating brush parts using this motor at the time of brushing to stimulate a scalp and promote blood flow.

In addition to the hairbrush device with the above-described structure, there are proposed a hairbrush-type massage device and the like which, when patting a scalp with a plurality of elastic prongs equivalent to brush parts, uses deformation occurring on the elastic prongs so that tip portions of the elastic prongs are moved in a radial direction and gives a massage action (for example, see Japanese Patent Laid-open Application No. Hei 9-122192).

However, in the vibration type hairbrush device described in the former patent document, a hair growth effect obtained by stimulating the scalp by vibration is not necessarily sufficient, and improvement in the hair growth effect is sought. Also in the device in the latter patent document, from a function such as using elastic deformation by a compressive force to slightly move the tip portions of the elastic prongs in the radial direction, a good massage effect or an effect of removing dirt from the scalp cannot be expected much, and thus the device has problems in this regard.

Additionally, in the device used for such a purpose, it is desired to add, besides being the device for hair growth, a function of, for example, alleviating shoulder stiffness or muscle ache, or further, a function such as, for example, performing skin beautification on a body part such as a hand/foot, shoulder, face, or scruff of the neck.

Here, when an environment in which the above-described hair growth treatment or treatment to body parts is actually performed is considered, it is desired to perform the above-described treatment also in the environment such as in a bathroom so that shampooing or cleaning of a body part can be done at the same time.

### Disclosure of the Invention

The present invention is made to solve such problems, and its object is to provide a treatment device enabling to obtain a superior hair growth effect by massage to a scalp or removing of dirt from the scalp being performed preferably, and also enabling to perform massage or skin beautification to a body part, and further, enabling to perform treatment without worrying about penetration of water and the like to the device even in a usage environment such as in a bathroom.

To achieve the above object, a treatment device of the present invention includes: a hous ing in which an electric component including a power supply is incorporated; a contact projected on the housing and used with a tip portion being contacted with a skin surface of an object to be treated; a waterproofing sealing member which seals an accommodating portion for the electric component in the housing against the outside; and a contact drive mechanism which reciprocates the contact the tip portion of which is contacted with the skin surface of the object to be treated in a direction along the skin surface.

The treatment device of the present invention also includes first and second contact groups respectively by providing a plurality of the contacts respectively, and the contact drive mechanism reciprocates the first and second contact groups respectively in a manner that approach or separation of a tip part of the first contact group and a tip part of the second contact group is repeated.

Further, in the treatment device of the present invention, as for the first and second contact groups, the plural contacts of the first contact group and the plural contacts of the second contact group are arranged zigzag to evade intersection of movement trajectories of the contacts.

In the treatment device of the present invention, the housing is constituted with a first casing having the accommodating portion for the electric component and a second casing supporting the contacts, and the treatment device of the present invention further includes an attachment/detachment mechanism to join the second casing in relation to the first casing attachably/detachably.

Further, in the treatment device of the present invention, the contacts are a brush.

The treatment device of the present invention further includes a light irradiation mechanism which irradiates light of a certain wavelength region to the skin surface of the object to be treated.

Further, in the treatment device of the present invention, the contact is formed of a translucent material.

The treatment device of the present invention includes: a light irradiation mechanism which irradiates light of a certain wavelength region to a skin surface of an object to be treated; a housing in which the light irradiation mechanism and an electric component including at least a power supply are incorporated; a waterproofing sealing member which seals an accommodating portion for the electric component in the housing against the outside; first and second brush groups made by making a plurality of brushes projected on the housing into units respectively, the brushes having translucency transmitting the light emitted by the light irradiation mechanism and being used with tip portions contacted with the skin surface of the object to be treated; and a brush drive mechanism which reciprocates the first and second brush groups respectively in a direction along the skin surface in a manner that approach or separation of a tip part of the first brush group and a tip part of the second brush group tip portions of which are contacted with the skin surface of the object to be treated is repeated.

Further, in the treatment device of the present invention, as for the first and second brush groups, the plural brushes of the first brush group and the plural brushes of the second brush group are arranged zigzag to evade intersection of movement trajectories of the brushes.

In the treatment device of the present invention, the housing in which the light irradiation mechanism and an electric component including at least a power supply are incorporated is constituted with a first casing having the accommodating portion for the electric component and a second casing supporting the respective brushes, and the treatment device of the present device further includes an attachment/detachment mechanism tojoin the second casing in relation to the first casing attachably/detachably.

### Brief Description of Drawings

Fig. 1 is a perspective view showing a treatment device of a first embodiment of the present invention.
Fig. 2 is an disassembled perspective view of the treatment device shown in Fig. 1.
Fig. 3 is a perspective view of the treatment device shown in Fig. 1 seen from a base end portion side.
Fig. 4 is across-sectional view showing a brush drive mechanism (separation state of brushes) which the treatment device of Fig. 1 includes.
Fig. 5 is a cross-sectional view showing an approached state of the brushes of the brush drive mechanism of Fig. 4.
Fig. 6 is a plan view of the brush drive mechanism of Fig. 4 seen from a tip side of the brush.
Fig. 7 is a perspective view of a treatment device of a second embodiment of the present invention seen from a base end portion side.
Fig. 8 is a cross-sectional view showing a structure of first and second brush units and a periphery thereof which the treatment device of Fig. 7 includes.
Fig. 9 is a cross-sectional view showing another brush drive mechanism (separation state of brushes) whose structure is different from that of the mechanism of Fig. 4.
Fig. 10 is a cross-sectional view showing an approached state of the brushes of the brush drive mechanism of Fig. 9.

### Best Mode for Carrying out the Invention

Hereinafter, the present invention will be described in detail according to the attached drawings.

### (First Embodiment)

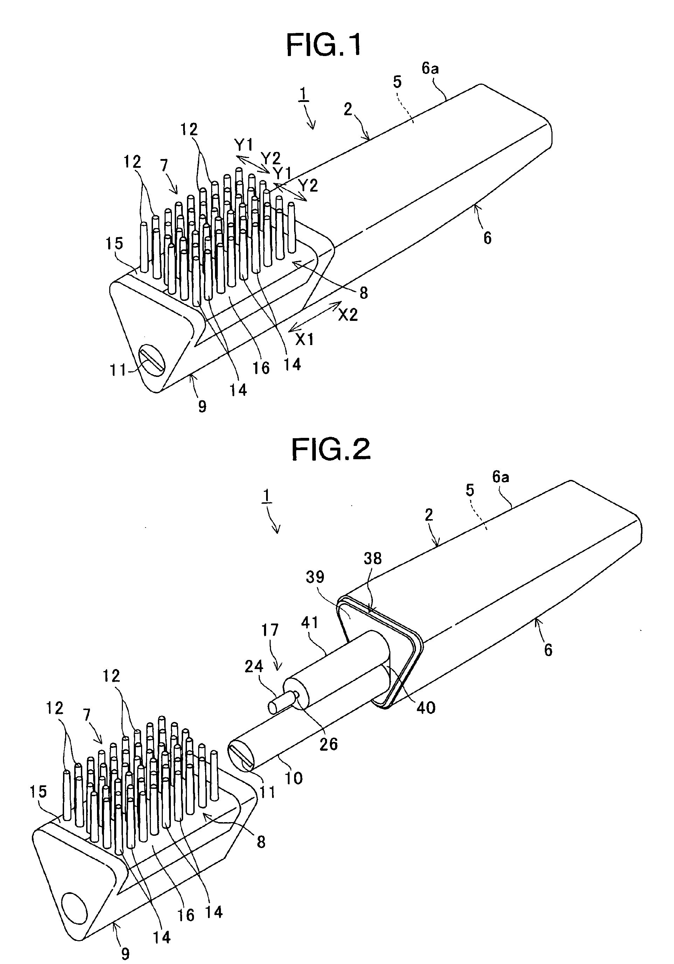

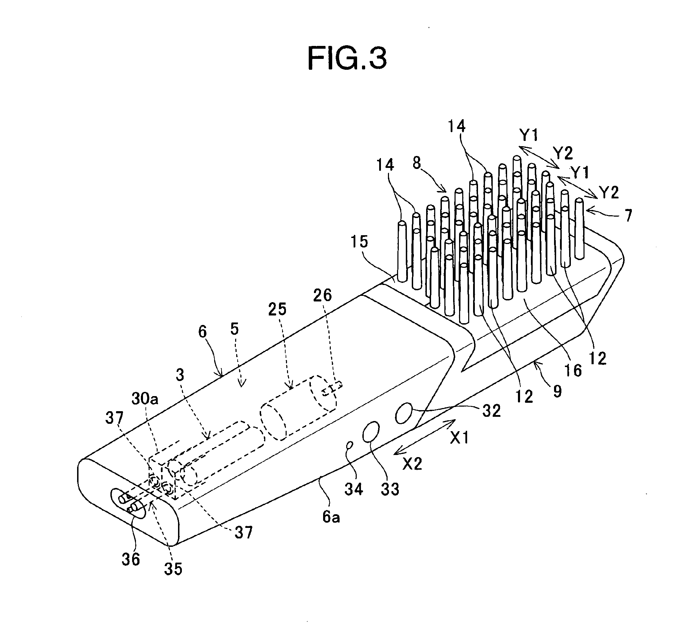

Fig. 1 is a perspective view showing a treatment device of a first embodiment of the present invention, Fig. 2 is an disassembled perspective view of this treatment device, and Fig. 3 is a perspective view of this treatment device seen from a base end portion side.

As shown in these drawings, this treatment device 1 is a handy type (portable) device allowing a user himself to perform hair growth treatment to a scalp and treatment such as massage to a body parts such as a hand/foot or shoulder or skin beautification. An outer shell of the treatment device 1 is formed of a housing 2. The housing 2 is constituted with a first casing 6 having an accommodating portion 5 for an electric component including a rechargeable battery 3 as a power supply, and a second casing 9 supporting both a first brush unit 7 and a second brush unit 8 as first and second contact groups. To the first casing 6, a grip portion (handle) 6a to be gripped by the user is provided.

The second casing 9 which is formed into a substantially triangular prism shape is constructed to be attachable/detachable in relation to the first casing 6. That is, as shown in Fig. 2, to the first casing 6, a column-shaped lock bar 10 projecting in a direction of a tip of the first casing 6 is provided. On an end surface of a most tip portion of the lock bar 10, an operation groove 11 is formed, and in the lock bar 10, the lock bar 10 main body can be slightly pivoted in a direction along a peripheral surface thereof (peripheral surface of the column shape) via this operation groove 11.

Additionally, on a predetermined position of the peripheral surface of the lock bar 10, an engaging portion is provided. This engaging portion is formed in a manner to be engaged with a portion to be engaged which is provided, for example, on an inner wall part of the second casing 9. The engaging portion of the lock bar 10 is locked with the portion to be engaged on the inner wall of the second casing 9 when an engaging shaft(lock bar) 10 is pivoted by a certain pivot angle or more from a predetermined position in a state that the second casing 9 is disposed serially in relation to the first casing 6. That is, the lock bar 10 of the first casing 6 side having the operation groove 11 and the engaging portion, and the portion to be engaged on the inner wall of the second casing 9, function as an attachment/detachment mechanism to join the second casing 9 in relation to the first casing 6 attachably/detachably.

Hereby, it becomes possible to detach the second casing 9 having the first and second brush units 7, 8, which, for example, have become dirty due to repeated treatment, from the first casing 6 side having the electric component, in a direction of an arrow X1 - X2 and to clean it. Therefore, it is possible to effectively perform after-described hair growth treatment and the like with the first and second brush units 7, 8 which are always in a clean state.

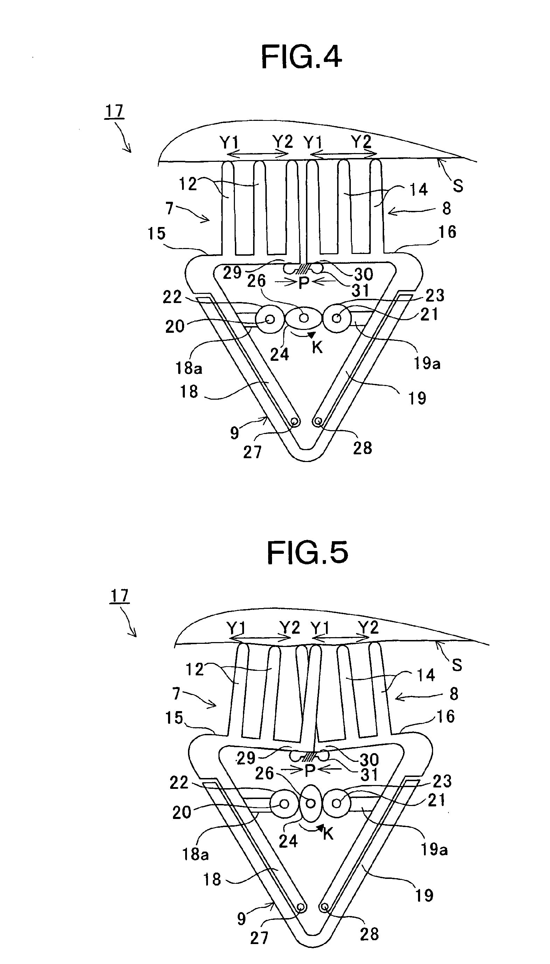

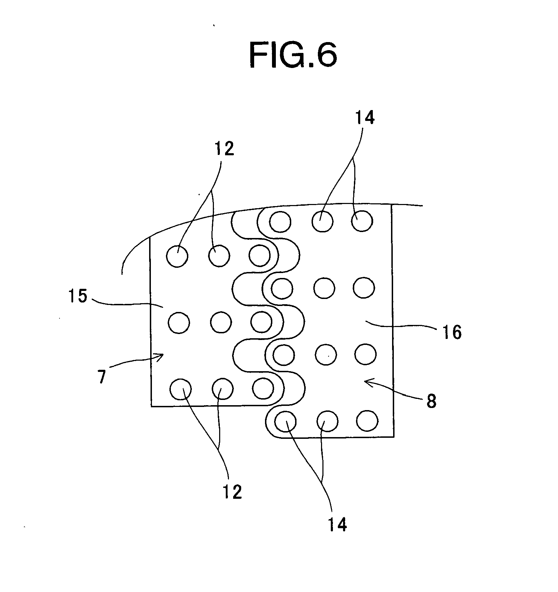

Next, a brush drive mechanism as a contact drive mechanism provided to the treatment device 1 of the present embodiment will be described using Fig. 4 to Fig. 6 in addition to Fig. 1 to Fig. 3. Here, Fig. 4 is a cross-sectional view of the brush drive mechanism in which the first and second brush units 7, 8 respectively are in a separate state, Fig. 5 is a cross-sectional view of the brush drive mechanism in which the first and second brush units 7, 8 respectively are in an approached state, and Fig. 6 is a plan view of the brush drive mechanism seen from a tip side of the brush.

As shown in these drawings, the first and second brush units 7, 8 are constructed in a manner that brushes 12, 14 being a plurality of, for example about respectively thirty, contacts are integrally supported on brush supporting portions 15, 16 via base end portions of the brushes 12, 14. That is, the plural brushes 12, 14 are projected in relation to the housing 2 (second casing 9). The individual brushes 12, 14 are formed of a resin material or the like in column shapes with spherical portions provided on tips thereof.

The brush drive mechanism 17 reciprocates, that is, slides consecutively, the brushes 12, 14 respectively ,the tip portions of which are contacted with a skin surface S of an object to be treated in a direction along the skin surface S. Specifically, the brush drive mechanism 17 reciprocates the first and second brush units 7, 8 in directions of arrows Y1 - Y2 (reciprocatingly displaces in a radial direction of the brush) in a manner that approach or separation of a tip part of the brush 12 of the first brush unit 7 and a tip part of the brush 14 of the second brush unit 8 is repeated.

That is, the brush drive mechanism 17 includes, as shown in Fig. 4 and Fig. 5, first and second arms 18, 19 end portions of which are respectively connected to the brush supporting portions 15, 16 of the first and second brush units 7 , 8 , branch arms 18a, 19a diverging from central parts of the first and second arms 18, 19, first and second rollers 22, 23 respectively supported rotatably (in a manner to rotate freely) via supporting shafts 20, 21 provided to respective tip portions of the branch arms 18a, 19a, and an elliptical cum 24 or the like provided on a position in which a peripheral surface of the cum 24 slides against individual peripheral surfaces of the first and second rollers 22, 23, respectively. A central portion of this cum 24 is fixed to a drive shaft 26. This drive shaft 26 is coupled to an output shaft of a motor 25 accommodated in the accommodating portion 5 for the electric component in the first casing 6 via a coupling mechanism or the like.

Here, as for a unit made of the first brush unit 7 and the first arm 18, the other end portion of the first arm 18 to be a pivot center is supported pivotably (swingably in the direction of the arrow Y1 - Y2) by an arm shaft 27. On the other hand, as for a unit made of the second brush unit 8 and the second arm 19, the other end portion of the second arm 19 to be a pivot center is supported pivotably (swingably in the direction of the arrow Y1 - Y2) by an arm shaft 28. Respective base end portions of these arm shafts 27, 28 are supported on the second casing 9.

Further, on respective end portions facing each other of the brush supporting portion 15 and the brush supporting portion 16, hooking portions 29, 30 are provided, and between these hooking portions 29, 30, an extension spring 31 to generate a biasing force (pull strength) in directions of arrows P is provided. That is, this extension spring 31 biases the first and second arms 18, 19 in a direction in which the peripheral surfaces of the first and second rollers 22, 23 are pressure-contacted (pressed) on the peripheral surface of the elliptical cum 24.

Therefore, in the brush drive mechanism 17 constructed as above, when the motor 25 is supplied with power to drive the drive shaft 26 and the cum 24 is rotated in a direction of an arrow K, the first and second arms 18, 19 pivot (swing) with the arm shafts 27, 28 being pivot centers as shown in Fig. 4 and Fig. 5, and thereby the tip parts of the brushes 12, 14 of the first and second brush units 7, 8 are reciprocated in the directions of the arrows Y1 - Y2.

Additionally, as stated above, in the unit made of the first (or second) brush unit and the first (or second) arm, due to a structure in which the tip portion of the brush is largely apart from the pivot center part on the first (or second) arm, slight displacement of the arm in a vicinity of the cum 24 can become large displacement at the tip portion of the brush, so that a large slide (stroke) amount of the brush tip portion can be secured.

That is, in the treatment device 1, treatment can be performed while the brushes 12, 14 tip portions of which are contacted with a skin surface, for example, with a scalp, of the object to be treated are reciprocated. Therefore, according to the treatment device 1, due to a sliding action of the brush tip in relation to the scalp, it is possible to effectively remove dirt such as old corneum around a pore of the scalp or sebum (for example, over-oxidized lipid [peroxide] causing hair loss), and cleaning of the scalp can be enhanced.

In the treatment device 1, by a hair root part of the scalp cleaned as above being stimulated by a sliding with the brush tip, a metabolic function of hair is promoted and a superior hair growth effect can be obtained.

Further, in the treatment device 1, by the reciprocated brushes 12, 14 being pressed on the hand/foot, shoulder, waist or the like in which muscle fatigue or the like occurs, due to a massage effect brought by the part of the body being stimulated by a sliding action of the brushes 12, 14, shoulder stiffness, muscle pain or the like, for example, can be removed (alleviated) effectively.

According to the treatment device 1, by contacting the brushes 12, 14 with the hand/foot, scruff of the neck, face or the like of the object of skin beautification, due to a cleaning action of the skin surface brought thereby and a blood circulation promoting action or the like brought by massage performed by the brushes 12, 14 sliding on the skin surface, preferred skin beautification is performed.

Here, in the treatment device 1, as for the first and second contact groups, as shown in Fig. 6, the plural brushes 12 of the first brush unit 7 and the plural brushes 14 of the second brush unit 8 are arranged zigzag such that intersection (overlap) of movement trajectories of brushes are evaded three-dimensionally.

That is, implant positions of the plural brushes 14 constituting the second brush unit 8 are relatively staggered in a direction of the arrow X1 (or X2) in relation to implant positions of the plural brushes 12 constituting the first brush unit 7, and in such a manner these brushes 12 and brushes 14 are respectively arranged.

Hereby, in addition that miniaturization of the treatment device main body can be enhanced, the tip portions of the brushes 12, 14 can be slid densely against a predetermined region of the skin surface of the object to be treated, and an action of expanding and contracting the scalp by the sliding of the brush part can be obtained effectively, so that further improvement of the treatment effect can be enhanced.

Respective end portions facing each other of the brush supporting portion 15 and the brush supporting portion 16 are formed into projections and depressions and the projection and depression parts facing each other are arranged zigzag similarly to a layout of the brushes such that intersection of movement trajectories of the respective end portions at a time of reciprocation of the brush drive mechanism 17 can be evaded.

Next, a waterproof function provided in the treatment device 1 of the present embodiment will be described.

In the accommodating portion 5 for the electric component in the first casing 6 constituting the housing 2, there is incorporated the above-described rechargeable battery 3 which is provided as a driving power supply of the device main body and supplies electric power and the like to the motor 25, as shown in Fig. 3. Here, the treatment device 1 can be constructed in a manner that a dry-cell battery can be applied as the driving power supply, or a power induction circuit such as an AC (alternating current) power supply can be provided in the treatment device 1.

In a vicinity of the grip portion 6a of the first casing 6, a starter switch 32 and a stop switch 33 to turn on/off the driving power supply of the device main body are provided. In a vicinity of these switches, an LED 34 to show a power supply situation (on/off state of the power supply) by lighting/non-lighting thereof is provided.

Further, in the above-described accommodating portion 5 for the electric component in the first casing 6, there are accommodated a conductive member 30a towhich a terminal portion of the rechargeable battery 3 is connected and a charging connecter 35 a base end portion of which is connected to this conductive member 30a. The charging connector 35 is provided, as shown in Fig. 3, in a manner that a tip side part of the connector is exposed to the outside from a fit hole 36 for the charging adaptor and the like, the fit hole being provided on the base end portion of the housing 2 (first casing 6). Here, between the base end portion of the charging connector 35 and an inner wall part of the first casing 6 near the fit hole 36 which holds the base end portion of this connector, a waterproofing O-ring 37 is inserted.

As shown in Fig. 2, on the tip portion of the first casing 6, that is, on a step portion 38 where the first casing 6 and the second casing 9 are attached/detached, a triangular waterproofing packing 39 is inserted. In a central part of this packing 39, a long hole 40 is opened. A peripheral edge part of the long hole 40 is formed in a manner to stick fast to an outer peripheral part of a base end portion of the lock bar 10 which projects from the accommodating portion 5 for the electric component of the first casing 6 in a direction of a tip of the first casing 6 (the second casing 9 side), and to an outer peripheral part of a base end portion of a column-shaped guide bar 41 which guides the drive shaft 26 driving the cum 24, respectively.

Here, if a gap is generated between outer peripheral portions of the lock bar 10 and the guide bar 41, and a peripheral edge portion of the long hole 40, it is desirable to fill a resin or the like into this gap part. That is, the above-described O-ring 37 and the packing 39 function as waterproofing sealing members which seal the accommodating portion 5 for the electric component in the first casing 6 constituting the housing 2 against the outside.

Therefore, though the treatment device 1 includes above-described brushes 12, 14 which operate mechanically and the drive mechanism thereof (source of vibration which could cause penetration of water), it is possible to perform body cleaning or treatment for hair growth or the like without worrying about penetration of water and the like even in a usage environment such as in a bathroom. Hereby, preferred treatment can be performed together with shampooing or cleaning of the part of the body, for example.

As already described, in the treatment device 1 of the present embodiment, the tip parts of the brushes 12, 14 of the first and second brush units 7, 8, are reciprocated in a manner that approach or separation thereof is repeated. Therefore, according to the treatment device 1, if the object to be treated is, for example, the scalp or the like, while the action of practically expanding or contracting of the scalp is given by the brushes 12, 14 which are slid against the scalp, hair growth treatment can be performed.

Hereby, the effect of removing dirt from the scalp can be heightened, and activation of hair root cells and the like is enhanced by the above-described expansion and contraction (extension and contraction) action and the like of the scalp, so that preferred hair growth treatment can be performed.

Further, for this treatment device 1, there are exemplified various types of treatment such as a type of usage in which the tip parts of the brushes 12, 14 of the reciprocated first and second brush units 7, 8 are pressed on the scalp, a type of usage in which the scalp is patted with the tips of the reciprocated brushes 12, 14, and a type of usage in which hair is brushed with the reciprocated brushes 12, 14.

### (Second Embodiment)

Next, a second embodiment of the present invention will be described.

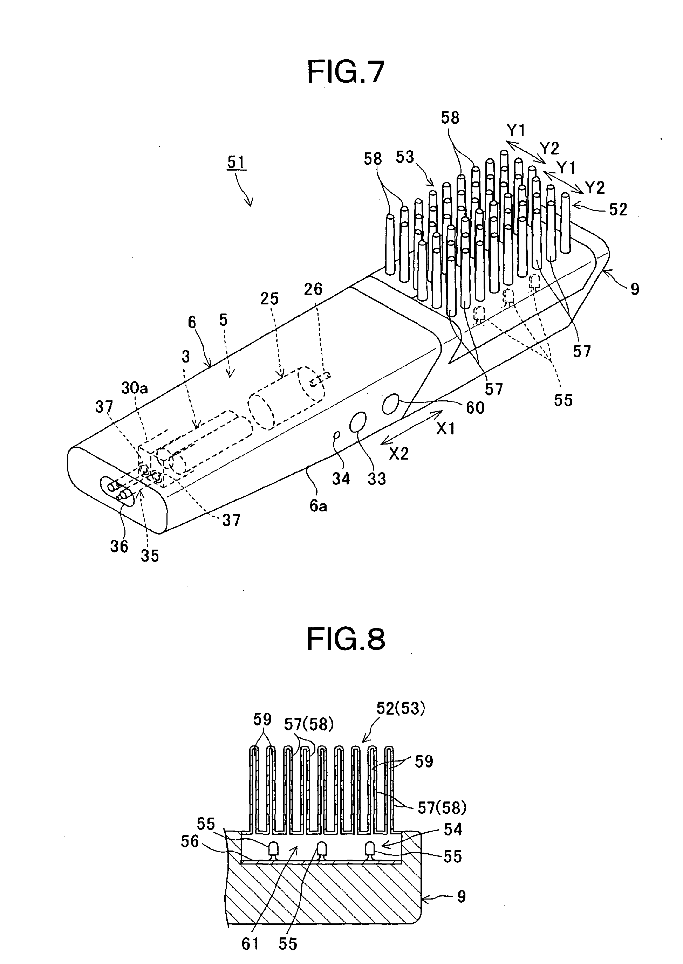

Fig. 7 is a perspective view of a treatment device according to the second embodiment of the present invention seen from a base end portion side, and Fig. 8 is a cross-sectional view showing a structure of first and second brush units and a periphery thereof which this treatment device includes.

As shown in these drawings, to a treatment device 51 of this embodiment, first and second brush units 52, 53 are applied instead of the first and second brush units 7, 8 which the treatment device 1 of the first embodiment includes, and further, a light irradiation mechanism 54 which irradiates light of a certain wavelength region is mounted.

The light irradiation mechanism 54 includes single or a plurality of LED lamp(s) 55 provided as (a) light source(s). The light irradiation mechanism 54 can include, in addition to the LED lamp(s) 55, various lenses, mirrors or the like to introduce light released from the LED lamp(s) 55 toward a skin surface S of an object to be treated (toward a brush tip side).

The light emitted by the LED lamp (s) 55 of the above-described light irradiation mechanism 54 is yellow light (for example, in a wavelength region from 572 to 575 nm), and has a luminous intensity of approximately 220 mcd, for example. Here, the light emitted by the LED lamp(s) 55 is optimal if it is light in the wavelength region from 572 to 575 nm as described above, or, it is desired the light is yellow light at least in a range from 550 to 580 nm.

Since this yellow light has high skin penetration, it is effective in removing a spot on a skin surface and so on. Additionally, as for a chromotherapy effect (action of color) by this yellow light, there are effects for restraining aging and skin roughness and so on.

As shown in Fig. 8, the LED lamp(s) 55 is (are) disposed in an internal space 61 between base end parts of a plurality of brushes 57, 58 constituting the first and second brush unit 52, 53 respectively and a main body part of a second casing 9, and is mounted on a base plate 56 attached to the second casing 9.

On the other hand, the first and second brush units 52, 53 respectively by providing a plurality of brushes 57, 58 are formed of a resin material having translucency (light transmission property) such as, for example, acrylic resin or polycarbonate. That is, in the treatment device 51, it is possible to irradiate the light emitted by the LED lamp(s) 55 of the light irradiation mechanism 54 to the skin surface of the object to be treated while making the light transmitted through the respective brushes 57, 58.

By this construction, it becomes possible to dispose the LED lamp(s) 55 inside the treatment device,51,as described above, so that a waterproofing effect of the LED lamp (s) 55 and an electric component in a periphery thereof can be obtained at a time such as using the treatment device 51 in a bathroom or the like.

Additionally, in central parts of the individual brushes 57, 58, there are respectively formed non-penetrating light guide holes 59 to guide the light emitted from the LED lamp (s) 55 to vicinities of the tip portions of the respective brushes 57, 58. These light guide holes 59 have openings in the above-described internal space 61 sides and are provided such that the light emitted from the LED lamp(s) 55 can be guided to tip portion sides of the individual brushes 57, 58 with loss of light amount as least as possible. The reason that the light guide holes 59 are non-penetrating in relation to the tip sides of the brushes 57, 58 is to secure a waterproofing property of the device interior.

Here, the treatment device 51 can be constructed, not by choosing the translucent material as the material for the first and second brush units 52, 53, but in a manner that (a) surface(s) of the LED lamp(s) 55 is (are) exposed between the base end portions of the individual brushes 57, 58, while a predetermined waterproofing function is secured.

In the treatment device 51 constructed as above, when a starter switch 60 is turned on to activate the device in a state that the tip portions of the brushes 57, 58 are contacted with the skin surface S of the object to be treated (for example, a scalp being an object of hair growth or a skin surface being an object to be beautified), the brushes 57, 58 repeat sliding operation in a direction along the skin surface S. Then, simultaneously, orange light is emitted from the LED lamp(s) 55, and this light is irradiated toward the skin surface S from the tip portions of the respective brushes via the light guide holes 59 of the respective brushes 57, 58.

Therefore, according to the treatment device 51 of the present embodiment, by a synergistic effect of an activation effect of tissue in a deep part of skin due to irradiation of light (yellow light) of a wavelength region (for example, from 572 to 575 nm) which is easy to be absorbed by hemoglobin, melamine or the like constituting the skin tissue to the skin surface S, a massage effect by the brushes 57, 58 being slid on the skin surface, and a vibration effect which can give active stimulation to the skin surface S at the time of sliding, preferred hair growth treatment or skin beautification can be performed. Specifically, since this yellow light irradiated to the skin surface S is easy to be absorbed by the above-described melamine, due to an evaporating effect in a pigment region, a skin whitening effect can be obtained.

Further, the treatment device 51 can be driven in a state that the scalp or skin surface is supplied with liquid chemical such as hair growth tonic or essence. In this case, relatively low-frequency vibration generated on the skin surface by a slide operation of the brush, and light of a certain wavelength region (orange light) act on the skin surface S, and thereby, aquaporin inside the skin opens, so that the liquid chemical can become easy to permeate into the skin.

Incidentally, though the above-described LED lamp(s) 55 emit(s) yellow light in the wavelength region from 572 to 575 nm, LED lamps can be applied which respectively emit, for example, blue light in a wavelength region from about 440 to 449 nm, green light in a wavelength region from 500 to 574 nm, orange light in a wavelength region from 595 to 640 nm, red light in a wavelength region from 641 to 700 nm, and the like. The above-described blue light is well absorbed by hemoglobin and effective against a reddish face or strawberry mark. Since the above-described green light has higher skin penetrance than the above-described blue light, the green light is absorbed by hemoglobin in a deep part of a dermis and is effective against a strawberry mark in the deep part of the dermis. Further, the above-described orange light is easy to be absorbed by melanin and hemoglobin and effective in removing a brown spot and so on. The above-described red light has high skin penetrance and reacts to melanin in the deep part of the skin, and is effective in removing a spot in the deep part of the skin.

Hereinbefore, the present invention is described concretely by the embodiments, but it is to be understood that the present invention is not intended to be limited to the above-described embodiments, and various changes may bemade therein without departing from the spirit thereof. For example, in the above-described embodiment, the brushes 12, 14 of the first and second brush units 7, 8 are to be reciprocated in the directions of the arrows Y1 - Y2, but instead, the brush drive mechanism can be constructed in a manner to reciprocate the plural brushes 12, 14 in the direction of the arrow X1 - X2.

In this case, it is desirable that the plural brushes 14 slide in the direction of the arrow X2 while the plural brushes 12 are sliding in the direction of the arrow X1. Hereby, an action of practically expanding or contracting, for example, the scalp surface or the like by, for example, a sliding with the brushes works in a sharing direction (alternately), so that the effect of removing dirt from the scalp or a massage effect can be heightened.

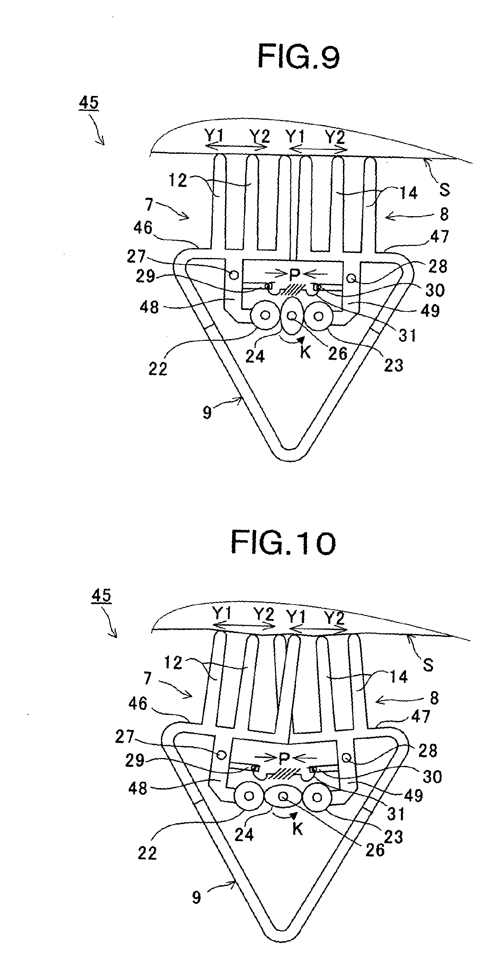

If a layout for disposing the above-described brush drive mechanism is restricted, instead of the above-described brush drive mechanism 17, a brush drive mechanism 45 can be applied as shown in Fig. 9 and Fig. 10. Here, in Fig. 9 and Fig. 10, the same symbols and numbers are given to almost the same members as component members of the brush drive mechanism 17 and explanation thereof will be refrained.

That is, the brush drive mechanism 45 includes first and second arms 48, 49 one end portions of which are respectively connected to the brush supporting members 46, 47. Second rollers 22, 23 are provided on the other end portions of the first and second arms 46, 47. An arm shaft 27 being a pivot center of a unit made of a first brush unit and the first arm 48 is provided on an almost central part of the first arm 48.

An arm shaft 28 being a pivot center of a unit made of a second brush unit and the second arm 49 is provided on an almost central part of the second arm 49. An extension spring 31 is hooked on hooking portions 29, 30 provided between the pivot center parts on the first and second arms 18, 19 and supporting portions of the first and second rollers 22, 23.

Also in the brush drive mechanism 45 with such a structure, the first and second arms 48, 49 pivot (swing) with the arm shafts 27, 28 being pivot centers, and hereby, tip parts of the brushes 12, 14 of the first and second brush units can be reciprocated in directions of arrows Y1 - Y2. Of course, the brush drive mechanism 45 exemplified in Fig. 9 and Fig. 10 can be applied to the treatment device 51 of the second embodiment.

### Industrial Applicability

A treatment device of the present invention can contact a reciprocated brush tip with a pressure point in a body having, for example, a slimming effect, and stimulate the pressure point by a sliding action of the brush or a vibration action generated at this time, and therefore it is possible to apply the treatment device as a slimming device and the like.

## Claims

1. A treatment device, comprising:
a housing in which an electric component including a power supply is incorporated;
a contact projected provided on said housing and used with a tip portion being contacted with a skin surface of an object to be treated;
a waterproofing sealing member which seals an accommodating portion for the electric component in said housing against the outside; and
a contact drive mechanism which reciprocates said contact the tip portion of which is contacted with the skin surface of the object to be treated in a direction along the skin surface.

2. The treatment device as set forth in claim 1,
wherein first and second contact groups respectively by providing a plurality of said contacts, and
wherein the contact drive mechanism reciprocates said first and second contact groups respectively in a manner that approach or separation of a tip part of said first contact group and a tip part of the second contact group is repeated.

3. The treatment device as set forth in claim 2,
wherein, in the first and second contact groups, the plural contacts of the first contact group and the plural contacts of the second contact group are arranged zigzag to evade intersection of movement trajectories of the contacts.

4. The treatment device as set forth in claim 1,
wherein the housing is constituted with a first casing having the accommodating portion for the electric component and a second casing supporting said contact, and
wherein an attachment/detachment mechanism to join the second casing in relation to the first casing attachably/detachably is provided.

5. The treatment device as set forth in claim 1,
wherein the contact is a brush.

6. The treatment device as set forth in claim 1, further comprising:
a light irradiation mechanism which irradiates light of a certain wavelength region to the skin surface of the object to be treated.

7. The treatment device as set forth in claim 1,
wherein the contact is formed of a translucent material.

8. A treatment device, comprising:
a light irradiation mechanism which irradiates light of a certain wavelength region to a skin surface of an object to be treated;
a housing in which the light irradiation mechanism and an electric component including at least a power supply are incorporated;
a waterproofing sealing member which seals an accommodating portion for the electric component in said housing against the outside;
first and second brush groups made by making a plurality of brushes projected on said housing into units, the brushes having translucency transmitting the light emitted by said light irradiation mechanism and being used with tip portions contacted with the skin surface of the object to be treated; and
a brushdrivemechanismwhich reciprocates the first and second brush groups respectively in a direction along the skin surface in a manner that approach or separation of a tip part of the first brush group and a tip part of said second brush group tip portions of which are contacted with the skin surface of the object to be treated is repeated.

9. The treatment device as set forth in claim 8,
wherein, in the first and second brush groups, the plural brushes of the first brush group and the plural brushes of the second brush group are arranged zigzag to evade intersection of movement trajectories of the brushes.

10. The treatment device as set forth in claim 8,
wherein the housing is constituted with a first casing having the accommodating portion for the electric component and a second casing supporting the respective brushes, and
wherein an attachment/detachment mechanism to join the second casing in relation to the first casing attachably/detachably is provided.

## Amended claims

### Amended claims under Art. 19.1 PCT

1. A treatment device, comprising:
a housing in which an electric component including a power supply is incorporated;
a contact projected on said housing and used with a tip portion being contacted with a skin surface of an object to be treated;
a waterproofing sealing member which seals an accommodating portion for the electric component in said housing against the outside; and
a contact drive mechanism which reciprocates said contact the tip portion of which is contacted with the skin surface of the object to be treated in a direction along the skin surface.

2. The treatment device as set forth in claim 1,
wherein first and second contact groups respectively by providing a plurality of said contacts, and
wherein the contact drive mechanism reciprocates said first and second contact groups respectively in a manner that approach or separation of a tip part of said first contact group and a tip part of the second contact group is repeated.

3. (Amended) The treatment device as set forth in claim 2,
wherein, the plural contacts of the first contact group and the plural contacts of the second contact group are respectively arranged in the state where each of the contacts is shifted in a direction orthogonal to a moving direction of the contacts to evade intersection of movement trajectories of the contacts.

4. The treatment device as set forth in claim 1,
wherein the housing is constituted with a first casing having the accommodating portion for the electric component and a second casing supporting said contact, and
wherein an attachment/detachment mechanism to join the second casing in relation to the first casing attachably/detachably is provided.

5. The treatment device as set forth in claim 1,
wherein the contact is a brush.

6. The treatment device as set forth in claim 1, further comprising:
a light irradiation mechanism which irradiates light of a certain wavelength region to the skin surface of the object to be treated.

7. The treatment device as set forth in claim 1,
wherein the contact is formed of a translucent material.

8. A treatment device, comprising:
a light irradiation mechanism which irradiates light of a certain wavelength region to a skin surface of an object to be treated;
a housing in which the light irradiation mechanism and an electric component including at least a power supply are incorporated;
a waterproofing sealing member which seals an accommodating portion for the electric component in said housing against the outside;
first and second brush groups made by making a plurality of brushes projected on said housing into units, the brushes having translucency transmitting the light emitted by said light irradiation mechanism and being used with tip portions contacted with the skin surface of the object to be treated; and
a brush drive mechanism which reciprocates the first and second brush groups respectively in a direction along the skin surface in a manner that approach or separation of a tip part of the first brush group and a tip part of said second brush group tip portions of which are contacted with the skin surface of the object to be treated is repeated.

9. (Amended) The treatment device as set forth in claim 8,
wherein the plural brushes of the first brush group and the plural brushes of the second brush group are respectively arranged in the state where each of the contacts is shifted in a direction orthogonal to a moving direction of the brushes to evade intersection of movement trajectories of the brushes.

10. The treatment device as set forth in claim 8,
wherein the housing is constituted with a first casing having the accommodating portion for the electric component and a second casing supporting the respective brushes, and
wherein an attachment/detachment mechanism to join the second casing in relation to the first casing attachably/detachably is provided.

11. (New) The treatment device as set forth in clam 6,
wherein the light irradiation mechanism irradiates light within a wavelength region from 550 to 580 nm.

12. (New) The treatment device as set forth in claim 7,
wherein the light irradiation mechanism comprises a light source in a base end portion side of the contact, and
wherein, in a central part of the contact, a non-penetrating light guide hole which has an opening only in the base end portion side of the contact and which is to guide the light emitted from said light source from the base end portion side to a tip portion side of said contact is formed.

13. (New) The treatment device as set forth in claim 2,
wherein the contact drive mechanism, comprising:
first and second arms respective one end portions of which are connected to the first and second contact groups and respective the other end portions of which are supported pivotably; and
a transfer mechanism for motive power including at least a cam which gives central parts of the first and second arms operating power to reciprocate said first and second contact groups, respectively.

14. (New) The treatment device as set forth in claim 8,
wherein the light irradiation mechanism irradiates light within a wavelength region from 550 to 580 nm.

15. (New) The treatment device as set forth in claim 8,
wherein the light irradiation mechanism comprises a light source in a base end portion side of the brush, and
wherein, in a central part of the brush, a non-penetrating light guide hole which has an opening only in the base end portion side of the brush and which is to guide the light emitted from said light source from the base end portion side of the brush to a tip portion side is formed.

16. (New) The treatment device as set forth in claim 8,
wherein the brush drive mechanism, comprising:
first and second arms respective one end portions of which are connected to the first and second brush groups and respective the other end portions of which are supported pivotably; and
a transfer mechanism for motive power including at least a cam which gives central parts of the first and second arms operating power to reciprocate the first and second brush groups, respectively.
